# EUROPEAN PATENT APPLICATION

(11) **EP 2 243 765 A1**
(43) Date of publication of application: **27.10.2010**
(21) Application number: 09005557.5
(22) Date of filing: 21.04.2009
(51) Int. Cl.: C07C 46/10, C07C 50/12

(54) **Recovery of vitamin K3 mother liquor**

(71) Applicant: Lonza Ltd., 4052 Basel (CH)
(72) Inventor: The designation of the inventor has not yet been filed

(57) **Abstract**

The present invention discloses a process for the recovery of Menadione from spent mother liquor containing a Menadione Bisulfite derivative, whereby the pH of the mother liquor is increased.

## Description

The present invention relates to a method for the recovery of Menadione (vitamin K3) from process mother liquors from any vitamin K3 derivative manufacturing process.

Water soluble salts of Vitamin K3 or Menadione are commonly used in the feed industry as the vitamin K active substances. The state of the art discloses several processes for the preparation of different vitamin K3 derivatives such as Menadione Sodium Bisulfite or MSB (US 2,367,302), Menadione dimethylprimidinol Bisulfite or MPB (US 3,328,169 and EP 0234940B1), Menadione Nicotinamide Bisulfite or MNB (UK 2025976A and BE 877.807) and other stabilized forms of vitamin K3 using adducts such as vitamin B6, p-Amino-Benzoic acid, etc (US 4,808,602 and US 4,577,019). In all these processes, once the liposoluble Menadione molecule is converted to a water soluble adduct using typically a bisulfite salt, the adduct is either precipitated as such (for example in the case of MSB) or it is put in contact with another protonated molecule that precipitates furthermore as a sulfonated salt of Menadione (such as MPB, MNB, etc). In all of these cases, the precipitation of the final salt is aided by way of cooling the mother liquor and/or concentrating the solution (using membrane treatment or evaporation techniques) and/or by way of adding a water miscible solvent and/or another water soluble salt that reduces the solubility of the Menadione sulfonated salt.

The main drawback of the state of the art is the loss of residual vitamin K3 in the final mother liquor after the precipitation step. The extent of the loss depends on the precipitation parameters affecting the maximal solubility of the vitamin K3 derivative in the final mother liquor solution. Examples of such parameters are temperature, nature of the water miscible solvent, presence of impurities, etc. The use of the water miscible solvent allows reaching lower residual concentrations of the vitamin K3 derivative in the spent mother liquor compared to the cooling step alone, but it involves additional steps of recycling the water miscible solvent for economic and environmental reasons.

Thus, there is a need to find a process which avoids the loss of vitamin K3 derivative in the mother liquor.

Said problem is solved by the process according to the present invention as defined in the claims.

It is well known that Menadione reacts with alkali metal bisulfites to form isomeric compounds with different anti-hemorrhagic activities. For example, the isomeric compounds of the Menadione reaction with sodium bisulfite are shown in the figure below:

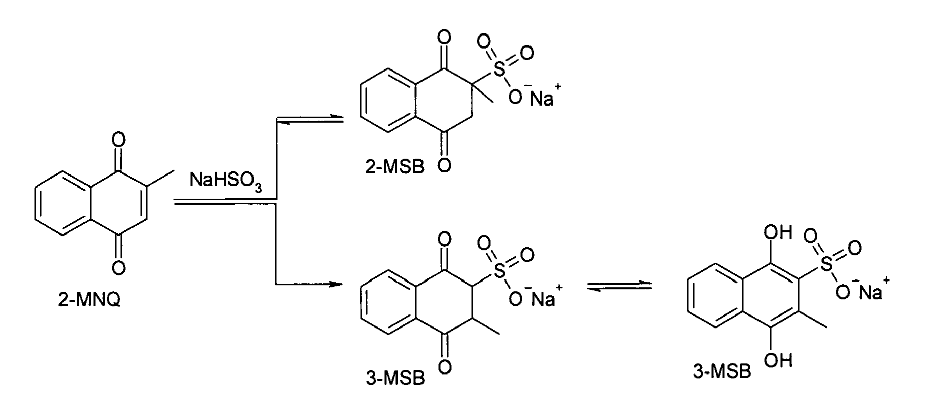

The 2-MSB isomer can produce free Menadione when brought in contact with an alkali solution following a β-cleavage reaction. The reaction is pH-dependent and also depends on the concentration of sodium bisulfite (J. C. Vire, G. J. Patriarche, G. D. Christian, Analyt. Chem. 1979, 51, 752-757). The β-cleavage reaction involves the following sequence of steps: removal of a proton at carbon-3, formation of the resonation enolate anion and spontaneous detachment of the sulfonate group as sulfite ion (R. T. Arnold, N. Bortnick, and E. McMullen, J. Am. Chem. Soc. 1942, 64, 1410-1413) as shown below:

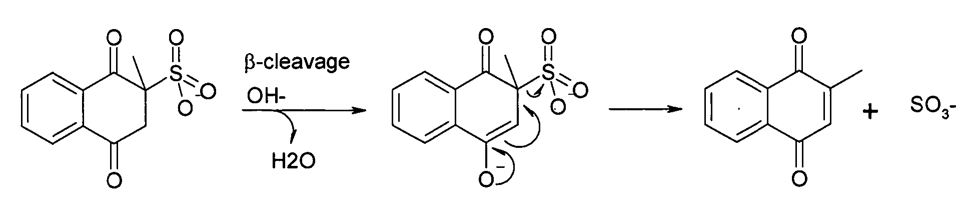

The 3-MSB isomer has approximately ten times less anti-hemorrhagic activity than the 2-MSB isomer (O. W. Charles, Feedstuff, 1972, vol. 44, No. 11, P. 35) and it yields only a reddish color in contact with alkali solutions (S. Yeh, G. A. Wiese, Drug Standard, 1958, 26, 22-29). In fact, since 3-MSB exists as its enol form in aqueous solution (thermodynamically more stable adduct, aromatization due to presence of enolizable hydrogen on carbon 3), no β -cleavage of a proton is possible.

The selective β-cleavage reaction of the 2-MSB isomer is the basis of the very efficient and economic way of recovering very pure Menadione from a spent mother liquor solution according to the present invention. The increase of the pH of a mother liquor (by adding an alkali solution) containing vitamin K3 bisulfite adduct will result in the formation of pure Menadione that is mostly insoluble in the aqueous solution. This reaction may be carried out both in a batch or a continuous mode.

However, there may arise the further problem that the presence of some ions in the mother liquor solution at certain concentrations interferes with the recovery method. Examples of such interfering ions are the bisulfite and sulfite ions that may exist in the solution as salts along with any inorganic or organic cations such as sodium, potassium, calcium, choline chloride, etc.

For example, in the case of sodium bisulfite solutions containing high concentrations of sodium bisulfite, it has been observed that the addition of alkali solutions does not result in the complete formation of free Menadione as expected.

This problem is due to the excess amounts of the bisulfite ions. Once they are eliminated from the solution, the conversion of the vitamin K3 bisulfite adduct to Menadione proceeds as expected. The elimination of excess bisulfite ions may be achieved by different means such as their conversion to sulfate ions, ion exchange treatment, membrane treatment or by their precipitation. Therefore, preferably, the β-cleavage reaction is carried out in the presence of metallic ions whose bisulfite or sulfite salts have a low solubility in water. Preferably, such metallic ions are selected from the group consisting of calcium, magnesium, strontium or barium. For example, the solubility of calcium sulfite, strontium sulfite and barium sulfite are 1 part in 800, 36,000 and 46,000 parts of water (F.P. Treadwell, Analytical Chemistry, Volume 1, Qualitative analysis, 1916, p. 349.), respectively, compared to the high solubility of sodium sulfite (which is 1 part in 3.2 parts of water).

The obtained Menadione precipitate may be separated from the original mother liquor solution by any typical means of separating a solid from the liquid such as by centrifugation or filtration.

In a preferred embodiment of the proposed method, the spent mother liquor solution is first put in contact with a water immiscible solvent before the pH is increased. Examples of the water immiscible solvents are Xylene, Toluene, Dichloroethane -or DCE, Dichloromethane or DMC, Chloroform, aliphatic solvents such as Hexane, Heptane, Octane, Nonane, Decane, etc.

The organic solvent containing the Menadione can be used for different applications. Examples are:
1. Production of pure vitamin K3
2. Production of other water soluble adducts (MSB, MPB, MNB, etc.)

### 1) Production of pure vitamin K3

Pure vitamin K3 may be obtained from this solution by means of known methods such as cooling of the solvent or by its evaporation. Compared to the Menadione obtained directly from the oxidation of 2-MethylNaphthalene, the Menadione obtained by this method is more pure due to the absence of the 6-methyl-1,4-naphthoquionone isomer formed during the oxidation reaction.

### 2) Production of vitamin K3 derivatives

The Menadione contained in the organic solvent may also be reacted with a bisulfite solution to produce a vitamin K3 bisulfite adduct. This reaction may be carried out in a batch or continuous mode. Once the Menadione is extracted from the water immiscible solvent, the solvent is separated from the vitamin K3 bisulfite adduct solution (e.g. by decantation, centrifugation, etc.) and it may be recycled back to the spent mother liquor treatment step for further recovery of Menadione. The vitamin K3 bisulfite adduct may then be precipitated from the aqueous solution by means of cooling or adding a water miscible solvent. Alternatively, the bisulfite adduct may be precipitated as stabilized forms of vitamin K3 as MNB, MPB, etc, by putting it in contact with well known compounds such as Nicotinamide, di-methyl-di-hydroxy-Pyrimidinol, etc. In all these cases, the spent mother liquor may be treated again with the proposed method to recover the residual amount of vitamin K3.

The present invention will be further described in the following, non-binding examples.

### Examples

### Example 1

20 parts of a spent mother liquor from a MNB precipitation reactor containing 0.11 M of Menadione sodium bisulfite and Nicotinamide was treated with 15 parts of a 10% sodium carbonate aqueous solution in the presence of 40 parts of chloroform. The mixture was agitated at room temperature and the two phases were separated. HPLC analysis of the mother liquor showed that 96,2% of the Menadione sodium Bisulfite had disappeared from the solution. GC analysis of the organic phase showed the presence of pure Menadione in the chloroform phase equivalent to the amount extracted from the mother liquor solution.

### Example 2

20 parts of the same spent mother liquor as in example 1 was treated with 0.8 parts of a 25% sodium hydroxide aqueous solution in the presence of 40 parts of chloroform. The mixture was agitated at room temperature and the two phases were separated. HPLC analysis of the mother liquor showed that 98.6% of the Menadione sodium Bisulfite had disappeared from the solution. GC analysis of the organic phase showed the presence of pure Menadione in the chloroform phase equivalent to the amount extracted from the mother liquor solution.

### Example 3

The same type of treatment as in example 1 was carried out on mother liquor solutions having the same range of MSB residual concentration of around 0,012-0,013 M and different residual amounts of sodium bisulfite. The results are presented in the table below.

| **Mother liquor composition (M)** | | **Mother liquor volume (parts)** | **Chloroform Volume (Parts)** | **CaCl₂ (6 M) Volume (parts)** | **NaOH 10% Volume (parts)** | **Menadione recovery yield** |
|---|---|---|---|---|---|---|
| **MSB** | **NaHSO₃** | | | | | |
| 0.0119 | 0 | 25 | 2 X 40 | 0 | 5 | 100 |
| 0.0121 | 2 | 25 | 2 X 40 | 0 | 15 | 65 |
| 0.0127 | 3.6 | 25 | 2 X 40 | 0 | 21 | 32 |
| 0.0126 | 3.6 | 25 | 2 X 40 | 15 | 21 | 59 |

As it may be seen, in the absence of any residual bisulfite ions in the mother liquor, the yield of the recovery of Menadione in the organic phase upon basification of the mother liquor solution is 100%. As the residual concentration of bisulfite ions increases to 2 M and 3,6 M in the spent mother liquor, the Menadione recovery yield is decreased to 65% and 32%, respectively. However, if calcium chloride is added to the mother liquor (as CaCl₂ in this example) before basification, the Menadione recovery yield is almost doubled from 32% to 59% for a mother liquor containing 3,6 M NaHSO₃

### Example 4

5 parts of a solution containing 0,34 M of Menadione sodium bisulfite was mixed with 5 parts of a 2 M NaHSO₃ aqueous solution to simulate a typical spent mother liquor. 2.5 parts of a CaCl₂ 4 M solution was added. The mixture was then treated with 2 parts of a 50% sodium hydroxide solution in the presence of 20 parts of chloroform. The mixture was agitated at room temperature and the two phases were separated. The analysis of the aqueous phases showed that around 56% of the initial NaHSO₃ had precipitated out. HPLC analysis of the mother liquor showed that 99% of the Menadione sodium Bisulfite had disappeared from the solution. GC analysis of the organic phase showed the presence of pure Menadione in the chloroform phase equivalent to the amount extracted from the mother liquor solution.

### Example 5

5 parts of a solution containing 0,326 M of Menadione sodium bisulfite was mixed with 5 parts of a 2 M NaHSO₃ aqueous solution to simulate typical spent mother liquor. 2.5 parts of a CaCl₂ 4M solution was added. The mixture was then treated with 2 parts of a 50% sodium hydroxide solution in the presence of 20 parts of Xylene. The mixture was agitated at room temperature and the two phases were separated. HPLC analysis of the mother liquor showed that 100% of the Menadione sodium Bisulfite had disappeared from the solution. GC analysis of the organic phase showed the presence of pure Menadione in the chloroform phase equivalent to 97,5% of the amount extracted from the mother liquor solution.

## Claims

1. Process for the recovery of Menadione from spent mother liquor containing a Menadione Bisulfite derivative, whereby the pH of the mother liquor is increased.

2. Process according to claim 1, whereby interfering ions are eliminated from the mother liquor prior to increasing the pH.

3. Process according to claim 2, whereby said elimination is effected by a method selected from the group consisting of selective precipitation, ion exchange treatment, membrane treatment or conversion into inert ions.

4. Process according to any of claims 1-3, whereby a water-immiscible solvent is added to the mother liquor prior to increasing the pH.

5. Process according to claim 4, whereby the water-immiscible solvent is selected from the group consisting of aromatic solvents such as Xylene or toluene, halogenated solvents such as chloroforme, dichloroethane or dichloromethane, or aliphatic solvents such as hexane, decane or cyclohexane.

6. Process according to any of claims 1-5, whereby the pH is increased by adding an alkaline solution.

7. Process according to claim 6, whereby the alkaline solution is selected from the group consisting of NaOH, KOH, Mg(OH)₂ or Ca(OH)₂.

8. Process according to any of claims 1-7, whereby the pH is increased to 12, more preferably to 11 and most preferably between 10,5 and 11.

9. Process according to any of claims 1-8, whereby the pH increase is carried out in the presence of metallic ions whose bisulfite or sulfite salts have a low solubility in water.

10. Process according to claim 9, whereby the metallic ions are selected from the group consisting of calcium and magnesium.

11. Process according to any of claims 1-10, whereby the Menadione Bisulfite derivative is selected from the group consisting of Menadione Sodium Bisulfite, Menadione dimethylprimidinol Bisulfite, Menadione Nicotinamide Bisulfite or other stabilized forms of vitamin K3.

12. Process according to claim 1, whereby the precipitated Menadione is separated from the mother liquor

13. Process according to claim 12, whereby the separation is carried out by centrifugation, filtration or sedimentation.

14. Process according to any of claims 1-13, whereby the process is carried out in a batch or continuous mode.
